# FASCICULE DE BREVET EUROPEEN

(11) **EP 3 968 881 B1**
(45) Date de publication et mention de la délivrance du brevet: **22.01.2025**
(21) Numéro de dépôt: 20754318.2
(22) Date de dépôt: 24.04.2020
(51) Int. Cl.: A61B 17/72

(54) **SYSTÈME DE FIXATION ENTRE UN DISPOSITIF MEDICAL ET AU MOINS UNE PARTIE D'UN OS**
BEFESTIGUNGSSYSTEM ZWISCHEN EINEM MEDIZINISCHEN GERÄT UND MINDESTENS EINEM TEIL EINES KNOCHENS
FIXATION SYSTEM BETWEEN A MEDICAL DEVICE AND AT LEAST ONE PORTION OF A BONE

(30) Priorité: 05.06.2019 FR 1905953
(43) Date de publication de la demande: 23.03.2022
(73) Titulaire: One Ortho, 69230 Saint-Genis-Laval (FR); Université Savoie Mont Blanc, 73000 Chambéry (FR)
(72) Inventeur: VACHER, Pierre, 74570 THORENS GLIERES (FR); GOYALLON, Thibault, 74000 ANNECY (FR); VITTECOQ, Eric, 74570 GROISY (FR); DODELIN, Arnaud, 76420 BIHOREL (FR); ARLETTAZ, Yvan, 18780 MONTHEY (CH); SCHENCK, Benoit, 67205 OBERHAUSBERGEN (FR); ANTONI, Maxime, 67000 STRASBOURG (FR); ALEPEE, Christophe, 69003 LYON (FR); DESPRES, Christophe, 74370 CHARVONNEX (FR)
(74) Mandataire: Cabinet Laurent & Charras
(86) Numéro de dépôt international: PCT/FR2020/050700
(87) Numéro de publication internationale: WO 2020/245515

(56) Documents cités:
- DE-U1- 202006 015 414
- US-A1- 2009 182 336

## Description

### DOMAINE TECHNIQUE

La présente invention se rapporte au secteur technique de la chirurgie orthopédique, et concerne plus particulièrement un système de fixation entre un dispositif médical et au moins une partie d'un os.

Par dispositif médical, on entend tout dispositif destiné à rester à long terme dans le corps d'un patient, tel qu'un implant de traumatologie, ou bien un implant orthopédique à tige prothétique, ou un clou centromédullaire par exemple.

### ETAT ANTERIEUR DE LA TECHNIQUE

Il est parfaitement connu pour un homme du métier d'utiliser différents dispositifs, communément appelés ancillaires de pose, pour le verrouillage et la fixation d'un implant sur un os, ou inversement pour la fixation de fragments osseux sur un implant.

Par exemple, pour l'enclouage centromédullaire, le clou centromédullaire présente une pluralité d'orifices de guidage, à positions angulaires et hauteurs prédéterminées, lesquels sont destinés à être traversés par des vis de fixation venant se visser dans la partie de l'os correspondante pour verrouiller en position le clou centromédullaire. Pour ce faire, le chirurgien dispose d'un ancillaire de pose, équipé d'un viseur externe permettant d'aligner un dispositif de perçage pour réaliser des orifices complémentaires dans la partie de l'os afin de pouvoir engager des vis de fixation au travers de l'os et du clou centromédullaire.

Les inconvénients de cette technique résident dans la nécessité d'utiliser, d'une part, un ancillaire de pose pour connaitre les axes de perçage et les axe des orifices destinés à recevoir les vis de fixation et, d'autre part, une radiographie de contrôle, pour vérifier le bon alignement et l'engagement correct des vis de fixation.

Cette technique est relativement complexe, et présente des risques pour le chirurgien de ne pas maîtriser l'alignement entre les perçages de l'os et les orifices du clou centromédullaire de sorte que les vis de fixation ne peuvent être engagées.

Il est également connu de l'homme du métier, pour remettre en position et en compression des fragments osseux sur un os, d'utiliser des sutures destinées à venir se nouer autour de l'os et des fragments osseux.

Cette technique présente l'inconvénient que les sutures ont tendance à réaliser une lacération vasculaire, laquelle provoque une nécrose par manque d'irrigation de l'os.

Il est connu de l'art antérieur, le document US2009/182336 fait apparaitre des mailles après déformation d'une structure dans un volume osseux donc il n'y a pas de maitrise de la taille des mailles et en plus une fois déformé la structure n'est plus extractible sur un malade.

Il est également connu le document DE202006015414 qui décrit un dispositif médical comprenant des vis et un implant intramédullaire, l'implant intramédullaire étant non expansible et présentant une paroi constituée de mailles définies par un treillis formé par une pluralité de brins rigides solidaires les uns des autres au niveau des intersections entre les différents brins, les mailles étant adaptées pour être traversées par la vis selon une position et une orientation non prédéfinies.

### EXPOSÉ DE L'INVENTION

L'invention est définie dans les revendications.

L'un des buts de l'invention est donc de remédier aux inconvénients précités en proposant un système de fixation entre un dispositif médical et au moins une partie d'un os, permettant éventuellement de s'affranchir de l'utilisation d'un ancillaire de pose ou d'un viseur externe, tout en permettant de réduire voire d'éviter l'utilisation de sutures et donc de réduire voire de supprimer le risque de nécrose.

À cet effet, et selon l'invention, il a été mis au point un système de fixation entre un dispositif médical et au moins une partie d'un os, remarquable en ce que le dispositif médical comprend une portion allongée, par exemple réalisée en polymère ou en métal biocompatible, destinée à être insérée dans l'os, notamment dans le canal médullaire et/ou dans les extrémités proximale ou distale. La portion allongée présente une paroi constituée de mailles, de préférence non-expansibles, adaptées pour être traversées par au moins un organe de fixation, selon une position et une orientation non prédéfinies, tout en exerçant par déformation élastique, plastique ou élasto-plastique, une force de pression, par exemple axiale et/ou transversale, sur ledit organe de fixation. L'organe de fixation est destiné à traverser également la partie de l'os pour assurer une fixation rigide entre l'os et le dispositif médical.

De cette manière, pour fixer entre eux une partie d'un os et le dispositif médical, l'invention propose d'utiliser un organe de fixation, tel qu'une vis, une pointe, ou une agrafe, éventuellement en combinaison avec un fil, et de faire traverser par cet organe de fixation, la partie de l'os à fixer et le dispositif médical. La portion allongée du dispositif médical, de préférence rectiligne, est notamment conçue pour être traversée par au moins un organe de fixation selon une position et une orientation non prédéfinies.

Ainsi, l'avantage de la présente invention réside dans le fait que l'organe de fixation traverse les mailles de la portion allongée du dispositif médical suivant une position non prédéfinie, que ce soit dans un plan diamétral ou excentré par rapport à l'axe longitudinal de la portion allongée, dans une direction orthogonale ou avec une incidence. De cette manière, le chirurgien peut choisir librement le nombre et la position des organes de fixation à utiliser, et peut choisir librement la technique de fixation de la partie osseuse, par exemple par agrafes, par vis, par pointes, etc. Par position et orientation non prédéfinie, il est entendu que le chirurgien peut lui-même décider, lors de l'intervention, de l'orientation et de la position de l'organe de fixation, il dispose d'une pluralité de choix pour l'orientation et le positionnement de l'organe de fixation.

Un autre avantage réside donc dans le fait que l'utilisation d'un ancillaire de pose ou d'une radiographie de contrôle n'est plus nécessaire, bien qu'en pratique, le chirurgien puisse tout de même l'utiliser pour retrouver l'orientation d'un organe de fixation déjà inséré.

Les mailles sont non-expansibles, c'est-à-dire qu'elles sont fixes et rigides de sorte qu'elles sont traversées par l'organe de fixation tout en exerçant, par déformation, une force de pression sur ledit organe de fixation. Cette force de pression est par exemple transversale et générée par déformation transversale des mailles de la portion allongée du dispositif médical. De cette manière, la force de pression transversale exercée sur l'organe de fixation permet de le maintenir en position et de le verrouiller et, par conséquent, de fixer la partie de l'os audit dispositif médical, ou bien de fixer le dispositif médical à l'os.

Par exemple, dans le cas d'un organe de fixation sous la forme d'une vis, la vis traverse la partie de l'os, et vient se visser à l'intérieur de la portion allongée du dispositif médical pour les fixer entre eux. Pour une fixation optimale, la vis traverse de part en part la portion allongée du dispositif médical et vient s'ancrer dans l'os.

Dans le cas d'un organe de fixation sous la forme d'une vis, les mailles présentent de préférence des épaisseurs sensiblement supérieures au pas de la vis, par exemple de quelques centièmes ou dixièmes de millimètres, pour exercer, par déformation axiale de l'épaisseur des mailles, une force de pression axiale sur la vis lorsqu'elle traverse les mailles.

Conformément à l'invention, les mailles sont définies par un treillis formé par une pluralité de brins rigides. Le treillis est par exemple réalisé en polymère ou en métal bio compatible, de préférence avec des brins à sections circulaires.

La portion allongée est par exemple tubulaire, et se présente avantageusement sous la forme d'une pièce réalisée par une technique d'impression en trois dimensions. Ainsi, il est aisé de réaliser le treillis avec des brins rigides à sections circulaires. Par ailleurs, les extrémités des brins sont reliées fixement entre elles. La technique d'impression en trois dimensions permet de réaliser facilement cette forme de réalisation et de maitriser parfaitement la dimension forme et la dimension des mailles pour ajuster parfaitement les forces de pression, transversale et/ou axiale, sur l'organe de fixation.

Afin d'éviter la croissance osseuse à l'intérieur de la portion allongée, cette dernière est revêtue d'un film ou remplie d'un matériau qui ne s'oppose pas au passage des organes de fixation mais qui interdit la croissance osseuse.

Selon des formes de réalisation différentes, le dispositif médical est constitué entièrement par la portion allongée elle-même, destinée à être insérée dans le canal médullaire de l'os et/ou à être logée dans l'extrémité proximale ou distale de l'os, pour y fixer des fragments d'os après immobilisation sur une partie de l'os sain.

Le dispositif médical peut également être un clou centromédullaire, ou une tige prothétique, comprenant sur leur partie destinée à être insérée dans le canal médullaire de l'os, la portion allongée selon l'invention, pour être traversée par des organes de fixation et verrouiller en position ledit clou centromédullaire ou la tige prothétique par rapport à la partie de l'os sain dans lequel ils sont implantés.

Le dispositif médical peut également être un clou centromédullaire, ou une tige prothétique, comprenant sur leur partie destinée à être logée dans les extrémités proximale ou distale de l'os, la portion allongée selon l'invention, pour être traversée par des organes de fixation et fixer ainsi les fragments osseux sur ledit clou centromédullaire ou la tige prothétique.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées dans lesquelles :

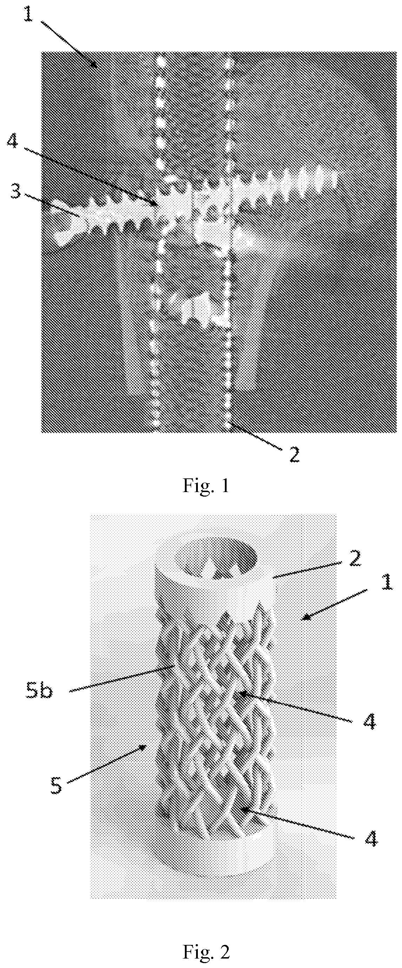

[Fig. 1] la figure 1 représente, en coupe longitudinale, un os avec la portion allongée d'un dispositif médical insérée dans le canal médullaire, et des vis permettant de fixer une partie de l'os sur le dispositif médical en venant se verrouiller à l'intérieur de la portion allongée ;
[Fig. 2] la figure 2 est une représentation schématique, illustrant en perspective, la portion allongée du dispositif médical avec une paroi constituée par un treillis formé d'une pluralité de fils tressés ;

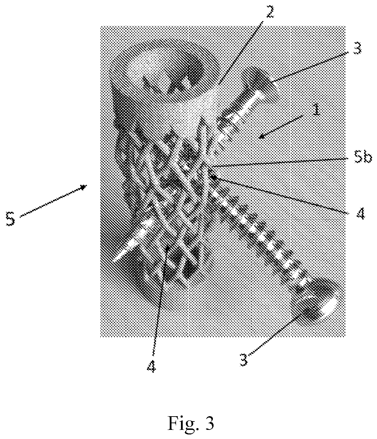

[Fig. 3] la figure 3 est une vue similaire à celle de la figure 2, illustrant également des vis de fixation traversant de, part en part, le treillis de la portion allongée.

### EXPOSE DETAILLE DE L'INVENTION

L'invention concerne un système de fixation entre un dispositif médical (1) et au moins une partie d'un os. L'invention trouve, par exemple, une application avantageuse pour remettre en position et en compression des fragments osseux sur un os, mais également pour la fixation sur un os de tout type de dispositif médical (1) destiné à être inséré dans le canal médullaire d'un os et/ou à être logé dans l'extrémité proximale ou distale de l'os.

À cet effet, selon l'invention, le dispositif médical (1) destiné à être fixé à un os, ou bien sur lequel des fragments osseux sont destinés à être fixés, comprend une portion allongée (2) destinée à être insérée dans le canal médullaire de l'os et/ou à être logée dans les extrémités proximale ou distale.

La portion allongée (2) peut soit constituer le dispositif médical (1) lui-même, lorsqu'il s'agit de venir y fixer des fragments osseux, soit constituer une partie d'un dispositif médical (1), par exemple une partie d'une tige prothétique ou bien d'un clou centromédullaire, lesquels sont destinés à être insérés dans le canal médullaire et fixés à l'os.

À cet effet, la portion allongée (2), de préférence tubulaire, présente une paroi constituée de mailles (4), non expansibles, adaptées pour être traversées, de part en part, par un ou plusieurs organes de fixation (3), tels que des vis, des agrafes, des pointes, etc., selon une position et une orientation non prédéfinies, et en exerçant, par déformation, une force de pression, notamment transversale et/ou axiale, sur lesdits organes de fixation (3).

Les mailles (4) sont définies par un treillis (5) formé par une pluralité de brins rigides (5b). Les brins rigides (5b) sont tressés et indépendants les uns des autres au niveau des intersections entre les différents brins (5b).

De cette manière, cela permet de pouvoir réaliser une portion allongée (2) rigide, non-expansible, avec des mailles (4) de dimensions maitrisées, tout en permettant de s'adapter à tout type d'organe de fixation (3), peu importe son orientation, puisque, étant donné que les brins (5b) sont indépendants les uns des autres au niveau des intersections, les brins peuvent s'écarter tout en continuant d'exercer la force de pression contre l'organe de fixation.

L'invention permet ainsi de fixer le dispositif médical (1) à une partie d'un os, ou bien des fragments osseux sur le dispositif médical (1), au moyen des organes de fixation (3) traversant l'os et la portion allongée (2) du dispositif médical (1).

L'invention permet au chirurgien de pouvoir choisir librement la position (hauteur, inclinaison et excentration) des organes de fixation (3), tout en pouvant s'affranchir de l'utilisation d'un ancillaire de pose ou d'une radiographie de contrôle. Les organes de fixation (3) peuvent traverser les mailles (4) de la portion allongée (2) suivant une position et une orientation non prédéfinies, c'est-à-dire pas forcément dans un plan diamétral, pas forcément perpendiculairement à l'axe de la partie allongée, au libre choix du chirurgien.

En pratique, lors de l'utilisation, un organe de fixation (3) traverse une partie de l'os puis traverse, de part en part, la portion allongée (2) en pénétrant successivement dans deux mailles (4), lesquelles exercent une pression, notamment transversale et/ou axiale, sur ledit organe de fixation (3), notamment du fait que l'une de leurs dimensions soit en ajustement serré avec l'organe de fixation (3). Par exemple, lorsque la largeur des mailles (4) est en ajustement serré, c'est-à-dire sensiblement inférieure de quelques dixièmes ou centièmes de millimètres par rapport à la section de l'organe de fixation (3), les mailles (4) exercent une pression transversale sur l'organe de fixation (3). Par exemple, lorsque l'organe de fixation (4) se présente sous la forme d'une vis, l'une des dimensions des mailles (4), notamment la largeur des mailles (4) peut être en ajustement serré avec le diamètre de fond de filet de la vis pour exercer une pression transversale sur la vis.

En alternative ou en combinaison, l'épaisseur des mailles (4) peut être en ajustement serré, c'est-à-dire sensiblement supérieure de quelques centièmes ou dixième de millimètres par rapport au pas de vis de sorte à exercer, par déformation, une force axiale sur la vis.

Selon une forme de réalisation particulière, la portion allongée (2) est réalisée, par exemple, à partir d'un grillage plan enroulé pour présenter une forme générale cylindrique, avec ou sans soudure axiale.

Une technique avantageuse consiste, en référence aux figures, à fabriquer, par fabrication additive, couche par couche, un tube dont une portion est réalisée par un treillis (5) avec une pluralité de brins rigides (5b), par exemple à sections circulaires.

Par ailleurs, les extrémités des brins (5b) sont reliées fixement entre elles aux deux extrémités de la portion allongée (2). La technique d'impression en trois dimensions permet de réaliser facilement cette forme de réalisation et de maitriser parfaitement la forme et la dimension des mailles (4) pour ajuster parfaitement les forces de pression transversale et/ou axiale, sur l'organe de fixation.

La géométrie des mailles (4) est adaptée à celle des organes de fixation (3). Cette géométrie permet de générer, par la déformation des mailles (4), une pression transversale et/ou axiale sur les organes de fixation (3), permettant de les verrouiller dans la portion allongée (2). Les efforts d'introduction desdits organes de fixation (3) doivent rester modérés, tout en assurant un assemblage ajusté, sans jeu, permettant une légère déformation de la maille (4).

À fin de faciliter le démontage, la portion allongée (2) peut être revêtue d'un film, non représenté, pour éviter la croissance osseuse à l'intérieur de ladite portion allongée (2), ou peut être remplie d'un matériau qui ne s'oppose pas au passage des organes de fixation (3) mais qui interdit la croissance osseuse.

Selon des formes de réalisation différentes, les mailles (4) peuvent être réalisées par un multi perçage au laser de la portion allongée (2), ou bien à partir d'un métal déployé, sans sortir du cadre de l'invention.

Il ressort de ce qui précède que l'invention fournit bien un système de fixation entre un dispositif médical (1) et au moins une partie d'un os, permettant de s'affranchir éventuellement de l'utilisation d'un ancillaire de pose ou d'un viseur externe. Pour réaliser la fixation, le chirurgien choisit le nombre et l'orientation des organes de fixation (3), en étant certain de pouvoir pénétrer la portion allongée (2) pour réaliser le verrouillage. L'invention permet de fixer des fragments osseux, tout en permettant de réduire voire d'éviter l'utilisation de sutures et donc de réduire voire de supprimer le risque de nécrose.

## Revendications

1. Dispositif médical (1) comprenant un système de fixation à au moins une partie d'un os, le système de fixation comportant au moins un organe de fixation (3), le dispositif médical (1) comprenant également une portion allongée (2) non expansible adaptée à être insérée dans l'os, notamment dans le canal médullaire et/ou dans les extrémités proximale ou distale de l'os, la portion allongée présentant une paroi constituée de mailles (4) définies par un treillis (5) formé par une pluralité de brins rigides (5b) tressés et indépendants les uns des autres au niveau des intersections entre les différents brins (5b) de sorte que les mailles (4) sont adaptées pour être traversées par l'au moins un organe de fixation (3), selon une position et une orientation non prédéfinies, tout en exerçant par déformation élastique, plastique ou élasto-plastique, une force de pression transversale et/ou axiale sur ledit organe de fixation (3), l'organe de fixation (3) étant adapté à traverser également la partie de l'os pour assurer une fixation rigide entre l'os et le dispositif médical (1).

2. Dispositif médical (1) selon la revendication 1*,* ***caractérisé* en ce que** le treillis (5) est réalisé avec des brins (5b) à sections circulaires.

3. Dispositif médical (1) selon l'une des revendications précédentes, ***caractérisé* en ce que** la portion allongée (2) est tubulaire.

4. Dispositif médical (1) selon l'une des revendications précédentes, ***caractérisé* en ce que** la portion allongée (2) est revêtue d'un film pour éviter la croissance osseuse à l'intérieur de ladite portion allongée (2) ou remplie d'un matériau qui ne s'oppose pas au passage des organes de fixation mais qui interdit la croissance osseuse.

5. Dispositif médical (1) selon l'une des revendications précédentes, ***caractérisé* en ce que** l'organe de fixation (3) est une vis.

6. Dispositif médical (1) selon la revendication 1*,* ***caractérisé* en ce que** l'une des dimensions des mailles (4) est en ajustement serré avec la section de l'organe de fixation (3) de sorte à exercer une pression transversale sur l'organe de fixation (3).

7. Dispositif médical (1) selon la revendication 5, ***caractérisé* en ce que** les mailles (4) présentent des épaisseurs en ajustement serré avec le pas de la vis de sorte à exercer une force de pression axiale sur la vis lorsqu'elle traverse les mailles (4).

8. Dispositif médical (1) selon l'une des revendications 1 à 7, ***caractérisé* en ce que** le dispositif médical (1) est un clou centromédullaire ou une tige prothétique.

9. Procédé de fabrication du dispositif médical de l'une des revendications précédentes, ***caractérisé* en ce qu'**il consiste à réaliser la portion allongée (2) par une technique d'impression en trois dimensions.

## Patentansprüche

1. Medizinisches Gerät (1), das ein Befestigungssystem an mindestens einem Teil eines Knochens umfasst, wobei das Befestigungssystem mindestens ein Befestigungselement (3) enthält. Das medizinische Gerät (1) umfasst zudem einen langgestreckten Abschnitt (2), der nicht expandierbar ist und so ausgelegt ist, dass er in den Knochen eingeführt werden kann, insbesondere in den Markkanal und/oder in die proximale oder distale Enden des Knochens. Der langgestreckte Abschnitt weist eine Wand auf, die aus Maschen (4) besteht, die durch ein Gitter (5) definiert sind. Das Gitter (5) wird aus einer Vielzahl von starren Fäden (5b) gebildet, die geflochten und an den Schnittpunkten der verschiedenen Fäden (5b) voneinander unabhängig sind, sodass die Maschen (4) dafür ausgelegt sind, von dem mindestens einen Befestigungselement (3) in einer nicht vorbestimmten Position und Orientierung durchquert zu werden, während sie durch elastische, plastische oder elastoplastische Verformung eine Quer- und/oder Axialdruckkraft auf das Befestigungselement (3) ausüben. Das Befestigungselement (3) ist so ausgelegt, dass es auch den Knochenteil durchquert, um eine starre Befestigung zwischen dem Knochen und dem medizinischen Gerät (1) zu gewährleisten.

2. Medizinisches Gerät (1) nach Anspruch 1, **dadurch gekennzeichnet, dass** das Gitter (5) aus Fäden (5b) mit kreisförmigem Querschnitt besteht.

3. Medizinisches Gerät (1) nach einem der vorhergehenden Ansprüche, **dadurch gekennzeichnet, dass** der langgestreckte Abschnitt (2) röhrenförmig ist.

4. Medizinisches Gerät (1) nach einem der vorhergehenden Ansprüche, **dadurch gekennzeichnet, dass** der langgestreckte Abschnitt (2) mit einer Beschichtung versehen ist, die das Knochenwachstum im Inneren des genannten langgestreckten Abschnitts (2) verhindert, oder mit einem Material gefüllt ist, das den Durchgang von Befestigungselementen nicht behindert, aber das Knochenwachstum verhindert.

5. Medizinisches Gerät (1) nach einem der vorhergehenden Ansprüche, **dadurch gekennzeichnet, dass** das Befestigungselement (3) eine Schraube ist.

6. Medizinisches Gerät (1) nach Anspruch 1, **dadurch gekennzeichnet, dass** eine Dimension der Maschen (4) eng an die Querschnittsabmessungen des Befestigungselements (3) angepasst ist, um einen seitlichen Druck auf das Befestigungselement (3) auszuüben.

7. Medizinisches Gerät (1) nach Anspruch 5, **dadurch gekennzeichnet, dass** die Maschen (4) Dicken aufweisen, die eng an die Gewindesteigung der Schraube angepasst sind, um eine axiale Druckkraft auf die Schraube auszuüben, wenn sie die Maschen (4) durchquert.

8. Medizinisches Gerät (1) nach einem der Ansprüche 1 bis 7, **dadurch gekennzeichnet, dass** das medizinische Gerät (1) ein intramedullärer Nagel oder eine Prothesenstange ist.

9. Herstellungsverfahren für das medizinische Gerät nach einem der vorhergehenden Ansprüche, **dadurch gekennzeichnet, dass** der langgestreckte Abschnitt (2) durch ein dreidimensionales Druckverfahren hergestellt wird.

## Claims

1. Medical device (1) comprising a fastening system to at least one part of a bone, the fastening system including at least one fastening element (3), the medical device (1) also comprising an elongated portion (2) that is non-expandable and designed to be inserted into the bone, particularly into the medullary canal and/or the proximal or distal ends of the bone, the elongated portion having a wall formed by meshes (4) defined by a lattice (5) made of a plurality of rigid strands (5b) woven and independent from one another at the intersections between the different strands (5b), such that the meshes (4) are configured to be traversed by the at least one fastening element (3), in a non-predefined position and orientation, while exerting, through elastic, plastic, or elasto-plastic deformation, a transverse and/or axial compressive force on said fastening element (3), the fastening element (3) being configured to also pass through the bone portion to ensure a rigid fixation between the bone and the medical device (1).

2. Medical device (1) according to claim 1, **characterized in that** the lattice (5) is made of strands (5b) with circular cross-sections.

3. Medical device (1) according to one of the preceding claims, **characterized in that** the elongated portion (2) is tubular.

4. Medical device (1) according to one of the preceding claims, **characterized in that** the elongated portion (2) is coated with a film to prevent bone growth inside said elongated portion (2) or filled with a material that does not impede the passage of fastening elements but prevents bone growth.

5. Medical device (1) according to one of the preceding claims, **characterized in that** the fastening element (3) is a screw.

6. Medical device (1) according to claim 1, **characterized in that** one dimension of the meshes (4) is in close fit with the cross-section of the fastening element (3) to exert transverse pressure on the fastening element (3).

7. Medical device (1) according to claim 5, **characterized in that** the meshes (4) have thicknesses in close fit with the thread pitch of the screw so as to exert an axial compressive force on the screw as it passes through the meshes (4).

8. Medical device (1) according to one of claims 1 to 7, **characterized in that** the medical device (1) is an intramedullary nail or a prosthetic rod.

9. Manufacturing method for the medical device of one of the preceding claims, **characterized in that** it involves creating the elongated portion (2) using a three-dimensional printing technique.
